# EUROPÄISCHE PATENTSCHRIFT

(11) **EP 3 237 114 B1**
(45) Veröffentlichungstag und Bekanntmachung des Hinweises auf die Patenterteilung: **12.06.2024**
(21) Anmeldenummer: 15820118.6
(22) Anmeldetag: 18.12.2015
(51) Int. Cl.: B02C 18/22, B02C 18/16

(54) **FESTSTOFF-AUSBRINGMODUL**
SOLIDS DISCHARGE MODULE
MODULE D'EXTRACTION DE MATIÈRE SOLIDE

(30) Priorität: 22.12.2014 DE 202014010155 U
(43) Veröffentlichungstag der Anmeldung: 01.11.2017
(73) Patentinhaber: Hugo Vogelsang Maschinenbau GmbH, 49632 Essen (DE)
(72) Erfinder: BURHORST, Torsten, 49681 Garrel (DE)
(74) Vertreter: Eisenführ Speiser
(86) Internationale Anmeldenummer: PCT/EP2015/080630
(87) Internationale Veröffentlichungsnummer: WO 2016/102394

(56) Entgegenhaltungen:
- DE-A1- 3 026 684
- DE-U1-202006 003 816
- US-A- 5 074 435
- US-A1- 2005 067 516

## Beschreibung

Die vorliegende Erfindung betrifft ein Feststoff-Ausbringmodul zum Abführen von Feststoffen aus Mischfluid führenden Vorrichtungen, insbesondere aus Zerkleinerungsvorrichtungen für Feststoffanteile in Mischfluid.

Die Erfindung betrifft insbesondere ein solches Ausbringmodul nach dem Obergriff von Anspruch 1. Die Erfindung betrifft ferner eine Vorrichtung nach dem Oberbegriff von Anspruch 14 sowie eine Verwendung eines Feststoff-Ausbringmoduls an den vorgenannten Vorrichtungen.

Mischfluide stellen die Hersteller entsprechender Vorrichtungen vor die Herausforderung, dass Mischfluide in sich sehr heterogen und von unterschiedlicher Beschaffenheit sein können. Neben verhältnismäßig hochviskosen Fluidanteilen oder Fluiden kann es auch zu einer Feststoffbeladung kommen, beispielsweise in Form Steinen, Metallpartikeln oder dergleichen.

Vorrichtungen zum Fördern von Mischfluid, wie beispielsweise Drehkolbenpumpen, besitzen aufgrund ihres Konstruktionsprinzips bereits eine hohe Toleranz für Feststoffbeladung. Nichtsdestotrotz sind die Feststoffe in den meisten Fällen unerwünschte Bestandteile im Fluidstrom. Die Feststoffanteile setzen sich entweder selbsttätig in bestimmten Sonderbereichen von Vorrichtungen ab, um aus dem Fluidstrom entfernt zu werden, oder werden in speziell vorgesehenen Feststoffabscheidern aus dem Fluidstrom getrennt.

Vorrichtungen zum Bearbeiten von Mischfluid, insbesondere Zerkleinerungsvorrichtungen wie beispielsweise die Produktreihe RotaCut der Hugo Vogelsang Maschinenbau GmbH werden in Anlagen zur Mischfluidförderung oder -behandlung in den Fluidstrom eingebunden, um darin mitgeförderte Feststoffanteile zu zerkleinern und für nachfolgende Bearbeitungsschritte des Mischfluids vorzubereiten, beispielsweise in der Lebensmittelproduktion oder der Biomasseverarbeitung. Auch aus solchen Anlagen ist es bekannt, dass bestimmte, besonders große und/oder schwere Feststoffanteile, in dafür vorgesehenen Sammelbereichen, beispielsweise mittels Sedimentation, aus dem Mischfluidstrom ausgeschieden werden.

Bei den vorstehend beschriebenen Vorrichtungen besteht trotz allgemein sehr zufriedenstellender Funktionsweise dennoch der Bedarf, die sich in der beschriebenen Weise ansammelnden Feststoffanteile zuverlässig und mit wenig Aufwand entfernen zu können.

Eine weitere Vorrichtung zum Zumischen von Flüssigkeiten und zum Fördern der erzeugten Suspensionen in eine Verarbeitungsanlage zeigt beispielsweise die DE 20 2006 003 816 U1. Die Vorrichtung weist einen Schneckenförderer, einen Feststoffabscheider sowie einen Zerkleinerer auf. DE 20 2006 003816 U1 schlägt vor, dass der Feststoffabscheider als geschlossener Behälter ausgebildet ist und alle an ihn angeschlossenen Bauteile nach außen hin abgeschlossen sind, nämlich die Förderschnecke, eine Feststoffschleuse, die Förderpumpe zum Zumischen von Flüssigkeit und der Zerkleinerer.

Dementsprechend lag der Erfindung die Aufgabe zugrunde, eine Möglichkeit bereitzustellen, diese Feststoffanteile möglichst effizient aus den vorgenannten Vorrichtungen entfernen zu können.

Die Erfindung löst die ihr zugrunde liegende Aufgabe in einem ersten Aspekt mit einem Feststoff-Ausbringmodul nach Anspruch 1. Das Feststoff-Ausbringmodul weist einen Feststoffeinlass, Koppelmittel zum Anschluss des Einlasses an eine Mischfluidförder- und/oder Bearbeitungsvorrichtung, einen Feststoffauslass, und einen Schließkörper, der an dem Feststoffauslass angeordnet und zwischen einer Freigabestellung und einer Sperrstellung hin- und herbewegbar ist, und eine sich von dem Feststoffeinlass zu dem Feststoffauslass erstreckende Förderkammer auf.

Gemäß der Erfindung weist das Feststoff-Ausbringmodul einen Füllstandsdetektor auf, der dazu eingerichtet ist, eine Feststoffansammlung in der Förderkammer zu erfassen und anzuzeigen. Der Füllstandsdetektor ermöglicht es auf einfache Weise, ohne den Betrieb der jeweils an das Ausbringmodul angeschlossene Vorrichtung unterbrechen zu müssen, die Überprüfung, ob sich in der Förderkammer eine ausreichende Menge Feststoff angesammelt hat, die das Öffnen des Schließkörpers zum Entfernen der Feststoffansammlung rechtfertigt oder erforderlich machen würde.

Gemäß einer bevorzugten Weiterbildung der Erfindung weist das Ausbringmodul ein Fördermittel auf, welches in der Förderkammer angeordnet und dazu eingerichtet ist, dem Feststoffeinlass zugeführte Feststoffe zum Feststoffauslass zu fördern. Durch das Fördermittel, welches erfindungsgemäß die ihm zugeführten Feststoffe aktiv weiterbefördert, wird eine Verstopfungsgefahr auf Seiten der angeschlossenen Förder- und/oder Bearbeitungsvorrichtung vermindert.

In einer bevorzugten Weiterbildung der Erfindung verläuft die Förderkammer unmittelbar stromaufwärts des Auslasses zur Horizontalen geneigt, insbesondere vertikal. Hierunter wird der Zustand verstanden, in dem sich das Feststoff-Ausbringmodul in betriebsgemäßer Orientierung befindet, also beispielsweise an eine Vorrichtung zum Fördern und/oder Bearbeiten von Mischfluid angeschlossenen Zustand.

Durch die geneigte Ausrichtung der Förderkammer zur Horizontalen rutschen oder fallen die angesammelten Feststoffe nach Bewegen des Schließkörpers in die Freigabestellung selbsttätig heraus, was die Gefahr eines Verstopfens des Ausbringmoduls auslassseitig minimiert.

In einer bevorzugten Ausführungsform der Erfindung ist der Schließkörper als Absperrschieber ausgebildet. Vorzugsweise ist der Schließkörper derart ausgerichtet, dass seine Bewegung von der Sperrstellung in die Ausbringstellung keine Verdrängung der angesammelten Feststoffe entgegen der Ausbringrichtung bewirkt. Bei Absperrschiebern, die keine Schwenkbewegung in oder entgegen der Strömungsrichtung beziehungsweise Ausbringrichtung zum Freigeben und Sperren vollziehen müssen, wird dies auf einfache Weise umgesetzt.

In einer weiteren bevorzugten Ausgestaltung ist der Füllstandsdetektor als Sonde ausgebildet, die einen Sondenkörper zum Einführen in die Förderkammer aufweist. Durch manuelles oder vorzugsweise auch automatisiertes Einführen des Sondenkörpers in die Förderkammer wird geprüft, ob der Sondenkörper beispielsweise bis zu einer maximalen Eindringtiefe in die Förderkammer eindringen kann, oder ob er vor Erreichen der maximalen Eindringtiefe auf ein Hindernis trifft. Im Zusammenhang mit der vorliegenden Erfindung wird eine Feststoffansammlung von ausreichender Menge also detektiert, indem der Sondenkörper nicht bis zu seiner maximalen Eindringtiefe vordringen kann, sondern bereits zuvor gegen die Feststoffansammlung stößt.

Besonders bevorzugt weist der Sondenkörper ein Anzeigemittel auf, welches von außen sichtbar und dazu eingerichtet ist, anzuzeigen, ob der Sondenkörper bis zu seiner maximalen Eindringtiefe vordringen kann oder nicht.

Weiter bevorzugt ist das Anzeigemittel dazu eingerichtet, die tatsächliche Eindringtiefe des Sondenkörpers anzuzeigen. Während grundsätzlich eine qualitative Aussage darüber, ob der Sondenkörper seine maximale Eindringtiefe erreichen kann, oder bereits zuvor auf ein Hindernis stößt, für einen zuverlässigen Betrieb der Vorrichtung ausreichend wäre, und mit Blick auf eine möglicherweise gewünschte besonders robuste Ausführung vorteilhaft wäre, kann es auch wünschenswert sein, anhand beispielsweise einer Skala ablesen zu können, wie weit der Sondenkörper eindringt, beziehungsweise um welchen Betrag der Sondenkörper seine maximale Eindringtiefe verfehlt, um den Grad der Ansammlung von Feststoffen stromaufwärts des Auslasses beurteilen zu können.

In einer weiteren bevorzugten Ausführungsform der Erfindung ist das Fördermittel als Volumenförderer ausgebildet. Vorzugsweise weist das Fördermittel einen Kolbenschieber auf, der von dem Feststoffeinlass in Richtung des Feststoffauslasses bewegbar und zwischen einer eingefahrenen Stellung und einer ausgeschobenen Stellung hin- und herbewegbar ist. Ein Kolbenschieber ist mechanisch robust herstellbar und mittels einer extern manuell oder wahlweise motorisch betätigbaren Schubstange ansteuerbar. Die Form des Kolbenkopfes und der umfänglichen Seitenflächen des Kolbenkopfes lassen sich präzise an eine Geometrie der Förderkammer anpassen, um ein jedenfalls für die auszubringenden Feststoffanteile unüberwindbares, also dichtes Anliegen an der Wand der Förderkammer zu gewährleisten. Da es nicht um absoluten Stoffaustrag, sondern in erster Linie um den Austrag von Feststoffanteilen geht, kann ein ausreichend kleiner Spalt zwischen Kolbenschieber und Förderkammerwand verbleiben, durch den beispielsweise flüssige Anteile, die mit abgeschieden werden, passieren können. Das passierende Fluid wird vorzugsweise mittels entsprechender Fluidein- und Fluidauslässe in dem Ausbringmodul zirkuliert oder aus dem Ausbringmodul abgeführt.

In einer bevorzugten, weil besonders robusten, Ausführungsform ist der Schließkörper manuell betätigbar. In einer bevorzugten alternativen Ausgestaltung, die dem Bestreben folgt, die Anzahl der manuell erforderlichen Eingriffe möglichst weitgehend zu reduzieren, ist der Schließkörper motorisch betätigbar. Unter motorischer Betätigbarkeit wird im Zusammenhang mit der Erfindung eine elektromagnetische, hydraulische oder pneumatische Aktuation verstanden, wobei diese jeweils entsprechend elektrisch ansteuerbar sind. Vorzugsweise weist das Feststoff-Ausbringmodul eine elektronische Steuereinheit auf, die signalleitend sowohl mit dem Füllstandsdetektor als auch mit dem entsprechenden Antrieb des Schließkörpers verbunden ist. Weiter vorzugsweise ist die elektronische Steuereinheit dazu eingerichtet, den Antrieb des Schließkörpers zu dessen Bewegung von der Sperrstellung in die Freigabestellung anzusteuern, beispielsweise mittels entsprechender Spannungssignale, wenn der Füllstandsdetektor mittels der signalleitenden Verbindung ein repräsentatives Signal dafür ausgibt, dass eine ausreichende Feststoffansammlung am Feststoffauslass vorliegt. Beispielsweise könnte der Füllstandsdetektor einen einfachen Spannungsschalter aufweisen, der zwei Schaltzustände unterscheidet; einen ersten Schaltzustand, in dem der Füllstandsdetektor keinen Schaltbedarf für den Schließkörper meldet, und einen zweiten Schaltzustand, in dem der Füllstandsdetektor einen Schaltbedarf für den Schließkörper meldet. Falls der Füllstandsdetektor als Sondenkörper ausgebildet ist, könnte das Erreichen der maximalen Eindringtiefe oder einer anderen vorbestimmten Eindringtiefe den ersten Schaltzustand repräsentieren, und ein Verfehlen der maximalen Eindringtiefe oder der anderen vorbestimmten Eindringtiefe könnte den zweiten Schaltzustand repräsentieren.

Die Erfindung löst die zugrundeliegende Aufgabe in einem weiteren Aspekt ferner durch ein Feststoff-Ausbringmodul zum Abführen von Feststoffen aus Mischfluid führenden Vorrichtungen, insbesondere aus Zerkleinerungsvorrichtungen für Feststoffanteile in Mischfluid, mit einem Feststoffeinlass, Koppelmitteln zum Anschluss an eine Mischfluid führende Vorrichtung, einem Feststoffauslass, einer sich von dem Feststoffeinlass zu dem Feststoffauslass erstreckenden Sammelkammer, und einem Füllstandsdetektor, der dazu eingerichtet ist, eine Feststoffansammlung in der Sammelkammer zu erfassen und anzuzeigen. Der Füllstandsdetektor ist vorzugsweise gemäß den vorstehend beschriebenen bevorzugten Ausführungsformen ausgebildet und macht sich dieselben Vorteile zunutze, weswegen diesbezüglich auf die obigen Darstellungen verwiesen wird. Es wird in diesem Aspekt bevorzugt, die Sammelkammer derart zur Horizontalen zu neigen (bezogen auf die betriebsbereite Ausrichtung des Ausbringmoduls), dass die durch den Feststoffeinlass in die Sammelkammer eintretenden Feststoffe selbsttätig, unterstützt durch die Schwerkraft, in Richtung des Feststoffauslasses rutschen oder fallen. Besonders bevorzugt wird die Sammelkammer als vertikales Sammelrohr oder als vertikaler Sammeltrichter ausgebildet.

Die Erfindung löst die ihr zugrunde liegende Aufgabe bei einer eingangs bezeichneten Mischfluid führenden Vorrichtung, indem die Vorrichtung einen Sammelbereich für aus dem Mischfluid abgeschiedene Feststoffe, und einen mit dem Sammelbereich kommunizierenden Auslass aufweist, wobei ein Feststoff-Ausbringmodul an den Auslass angeschlossen ist, welches nach einer der hierin beschriebenen bevorzugten Ausführungsformen ausgebildet ist.

Vorzugsweise sind bei jenen Vorrichtungen Koppelmittel zum Anschluss des Feststoff-Ausbringmoduls vorgesehen, die mit den Koppelmitteln des Feststoff-Ausbringmoduls kooperieren. Bevorzugt werden reversibel lösbare Koppelmittel, vorzugsweise zum kraft- oder formschlüssigen Verbinden des Ausbringmoduls mit der Vorrichtung. Unter den Vorrichtungen zum Bearbeiten von Mischfluid wird besonders bevorzugt, das erfindungsgemäße Ausbringmodul an eine Zerkleinerungsvorrichtung für Feststoffanteile in Mischfluiden anzuschließen.

Die vorstehend genannten Vorrichtungen weisen die gleichen bevorzugten Ausführungsformen auf und machen sich die gleichen Vorteile zunutze, wie sie bereits mit Bezug auf das erfindungsgemäße Feststoff-Ausbringmodul erörtert wurden. Insofern wird auf die obigen Ausführungen verwiesen.

Die Erfindung betrifft ferner eine Verwendung eines Feststoff-Ausbringmoduls. Die Erfindung löst die ihr zugrunde liegende Aufgabe, indem ein Feststoff-Ausbringmodul nach einer der hierin beschriebenen bevorzugten Ausführungsformen an einer Vorrichtung zum Fördern und/oder zum Bearbeiten von Mischfluid, insbesondere einer Zerkleinerungsvorrichtung für Feststoffanteile in Mischfluiden zum Entfernen von abgeschiedenen Feststoffen verwendet wird.

Die Erfindung wird im Folgenden unter Bezugnahme auf die beigefügten Figuren anhand bevorzugter Ausführungsbeispiele näher beschrieben. Hierbei zeigen:
- Figur 1: eine schematische räumliche Darstellung in Schnittansicht eines Feststoff-Ausbringmoduls gemäß einem ersten bevorzugten Ausführungsbeispiel,
- Figur 2: eine schematische räumliche Darstellung, teilweise im Schnitt, von einer Vorrichtung zum Bearbeiten und/oder Fördern von Mischfluid, mit einem Ausbringmodul gemäß Figur 1, und
- Figur 3: eine schematische räumliche Darstellung in Schnittansicht eines Feststoff-Ausbringmoduls gemäß einem zweiten bevorzugten Ausführungsbeispiel.

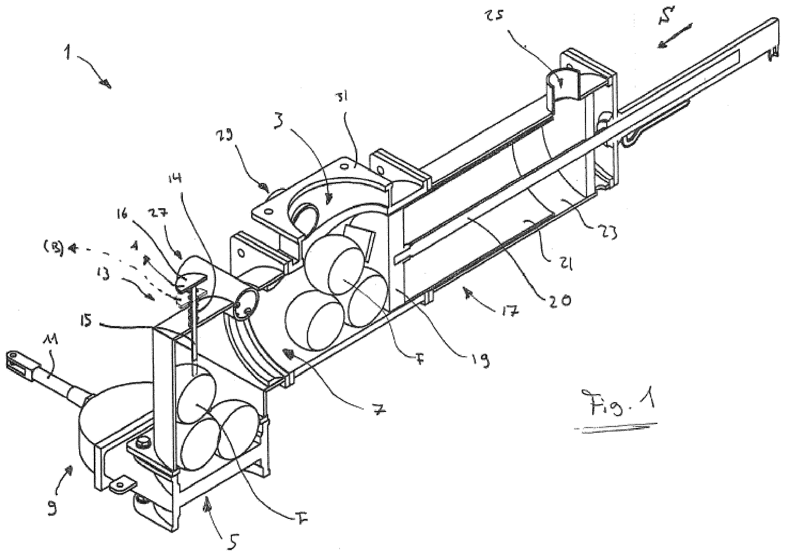

In Figur 1 ist ein Feststoff-Ausbringmodul 1 gemäß einem bevorzugten Ausführungsbeispiel der Erfindung dargestellt. Das Feststoff-Ausbringmodul 1 weist einen Feststoffeinlass 3, sowie einen Feststoffauslass 5 auf. Zwischen dem Feststoffeinlass 3 und dem Feststoffauslass 5 erstreckt sich eine Förderkammer 7. Die Förderkammer 7 weist stromabwärts des Einlasses 3 zunächst eine in angeschlossenem Zustand des Ausbringmoduls 1 horizontale Ausrichtung auf. In einem Endabschnitt stromaufwärts des Feststoffauslasses 5 ist die Förderkammer 7 zur Horizontalen geneigt, nämlich vertikal ausgerichtet.

Aus dem Feststoffauslass 5 ist ein Schließkörper 9 angeordnet. Der Schließkörper 9 ist vorliegend als Absperrschieber ausgebildet und mittels eines Betätigungselements 11 von der gezeigten Sperrstellung in eine Freigabestellung bewegbar, sowie wieder zurück.

In der Förderkammer 7 ist ein Füllstandsdetektor 13 für Feststoffanteile F angeordnet. Der Füllstandsdetektor 13 weist einen Sondenkörper 15 auf, an dem außerhalb der Förderkammer an einem Gehäuseteil des Ausbringmoduls 1 ein Tastkopf 16 angeordnet ist. Der Tastkopf 16 befindet sich in Figur 1 in einer festen, herausgedrückten Position A. Der Sondenkörper 15 ist gegen die Rückstellkraft einer Feder 14 in das Innere der Förderkammer 7 hineinbewegbar, und zwar so weit, bis er entweder seine maximale Eindringtiefe erreicht, oder aber bis er wie beispielsweise in der mit Pfeil B angedeuteten Stellung auf stromaufwärts des Feststoffauslasses 5 angesammelte Feststoffe F trifft.

Im Bereich des Feststoffeinlasse 3 ist in Figur 1 rechts neben dem Querschnitt des Feststoffeinlasses 3 ein Kolbenschieber 17 angeordnet. Der Kolbenschieber 17 weist einen Kolbenkopf 19 auf, welcher mittels einer Schubstange 20 durch die Förderkammer 7 in Richtung des Feststoffauslasses 5 bewegbar ist. Ein Kolbenmantel 21 erstreckt sich vom Kolbenkopf 19 aus entgegen einer Richtung S in die der Kolbenkopf 19 ausgehend von der Stellung in Figur 1 (zurückgezogene Stellung) in Richtung einer Ausschubstellung bewegt werden würde. Der Kolbenmantel 21 verschließt den Feststoffeinlass 3, wenn der Kolbenschieber 17 in der Richtung S durch die Förderkammer bewegt wird.

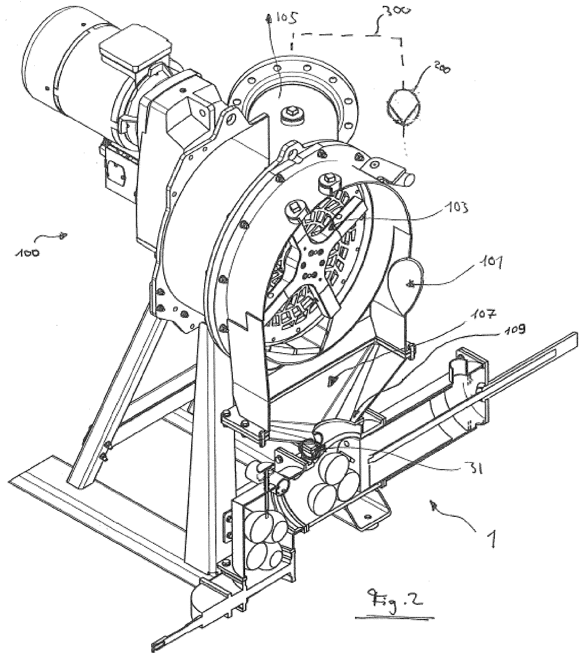

Der Kolbenmantel 21 ist der Wand 23 der Förderkammer 7 zugewandt und liegt entweder an ihr an, oder bildet mit der Wand 23 der Förderkammer 7 einen Spalt aus, der vorzugsweise so gering ist, dass Feststoffe nicht durch ihn hindurch können. Als Feststoffe sind hierbei diejenigen Partikel bzw. Gegenstände zu verstehen, welche in einer vorgeschalteten, über Kopplungsmittel 31 einlassseitig mit dem Ausbringmodul 1 verbundenen Mischfluid führenden Vorrichtung 100 (vgl. Figur 2) abgeschieden worden sind, weil sie zu groß und zu schwer sind, um dort im Fluidstrom zu verbleiben.

Es ist weiterhin ein Anschluss 25 vorgesehen, an den optional eine Leitung angeschlossen werden kann, die den Hohlraum zwischen dem Kolbenmantel 21 und der Wand 23 mit der Rohrleitung stromabwärts eines Schneidwerks 103 der Vorrichtung 100 verbindet. Dies hat den Vorteil, dass zwischen dem Kolbenkopf 19 und der Förderkammer 7 nicht abgedichtet werden muss.

Es wird durch den Vorgang des Austragens auch Flüssigkeit in Richtung des Feststoffauslasses 5 geschoben. Diese Flüssigkeit kann über den Anschluss 27 zu der Vorrichtung 100 zurückgeführt werden. Es findet durch die Schubbewegung ein Verdrängungsprozess statt. Damit der Druck nicht ansteigt, wird vorzugsweise die Flüssigkeit zurückgeführt.

Über einen Anschluss 29 kann Flüssigkeit wieder eingespült werden. Durch das Einspülen von Wasser an dieser Stelle soll das verbesserte Abscheiden von Fremdkörpern unterstützt werden.

Das Feststoff-Ausbringmodul 1 gemäß Figur 1 ist in Figur 2 in seiner Verwendung an einer Mischfluid führenden Vorrichtung 100 gezeigt. Die Vorrichtung 100 weist einen Mischfluideinlass 101 auf, ein Rotationsschneidwerk 103, und einen jenseits des Rotationsschneidwerks 103 angeordneten Mischfluidauslass 105 auf. Die Mischfluid führende Vorrichtung 100 ist mithin als Zerkleinerungsvorrichtung ausgebildet, wie sie beispielsweise unter der Produktserie "RotaCut" der Hugo Vogelsang Maschinenbau GmbH bekannt sind. Da eine Zerkleinerungsvorrichtung wie beispielsweise der in Figur 1 abgebildete Typ keine eigene Förderwirkung erzielt, wird der Mischfluid führenden Vorrichtung 100 vorzugsweise ein Fluidleitungssystem 300 nachgeschaltet, in dem zusätzlich eine Pumpe 200 angeordnet ist, beispielsweise eine Drehkolbenpumpe.

Die Vorrichtung 100 weist einen Sammelbereich 107 auf, in dem diejenigen Feststoffe abgeschieden werden, die zu groß und/oder zu schwer sind, um - mitgerissen vom Fluidstrom - durch das Rotationsschneidwerk 103 zerkleinert zu werden und durch den Fluidauslass 105 die Vorrichtung 100 wieder zu verlassen.

An einem am unteren Ende des Sammelbereiches 107 angeordneten Auslass 109 ist mittels entsprechender Koppelmittel 31 das Feststoff-Ausbringmodul 1 mit seinem Feststoffeinlass angeschlossen.

Im vorliegenden Ausführungsbeispiel ist exemplarisch eine Zerkleinerungsvorrichtung als Mischfluid führende Vorrichtung 100 abgebildet. Anstelle einer Zerkleinerungsvorrichtung sind erfindungsgemäß jedoch noch andere Mischfluid führende Vorrichtungen vorgesehen, die einen Behälter aufweisen, in dem sich Feststoffe absetzen, wie beispielsweise Schwergutabscheider, Feststoffdosierer, beispielsweise von der Hugo Vogelsang Maschinenbau GmbH bekannt unter der Bezeichnung QuickMix, Zweiwellenzerkleinerer, beispielsweise von der Hugo Vogelsang Maschinenbau GmbH bekannt unter der Bezeichnung XRipper, Tankwagen, oder Pumpenanschlüsse.

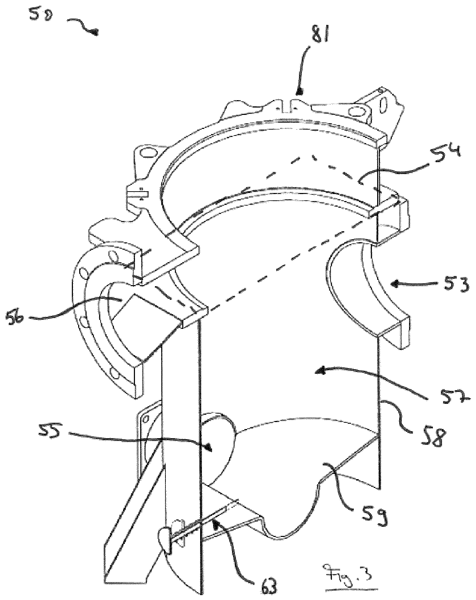

In Figur 3 ist ein Ausbringmodul gemäß einem zweiten Ausführungsbeispiel der Erfindung gezeigt. Das Feststoff-Ausbringmodul 50 gemäß Figur 3 weist anstelle eines Ausschiebemechanismus und einer Förderkammer eine, vorzugsweise im Betrieb vertikal ausgerichtete, Sammelkammer 57 auf. In der Sammelkammer 57 ist, in Figur 3 oben rechts, ein Mischfluid- und Feststoffeinlass 53 angeordnet. Oberhalb des Feststoffeinlasses 53 befindet sich eine Flanschebene 54, auf deren Höhe ca. das Schneidwerk 103 der Vorrichtung 100 in montierter Stellung sitzen kann. Oberhalb der Ebene 54 ist, in Figur 3 links, ein Flüssigkeitsauslass 56 stromabwärts der Ebene 54 für die Zerkleinerungsvorrichtung 103 ausgebildet. Das Ausbringmodul 50 weist an seiner Oberseite Koppelmittel 81 zum Anschluss an eine mischfluidführende Vorrichtung wie beispielsweise die Vorrichtung 100 aus Figur 2 auf.

Die Sammelkammer 57 ist im Inneren eines zylindrischen Rohrs 58 ausgebildet, an dessen unterem Ende ein Feststoffauslass 55, auch bezeichnet als Reinigungsöffnung, angeordnet ist. Der Feststoffauslass 55 ist optional mit einem Schließkörper verschließbar, beispielsweise ausgebildet als Absperrschieber wie im ersten Ausführungsbeispiel.

In Nähe des Bodens 59 der Sammelkammer 57 ist ein Füllstandsdetektor 63 angeordnet. Der Füllstandsdetektor 63 ist ebenfalls wie beim ersten Ausführungsbeispiel dazu eingerichtet, die Anwesenheit von Feststoffen am unteren Ende der Sammelkammer zu detektieren. Die Höhe der Anbringung des Füllstandsdetektors bestimmt den Zeitpunkt, zu dem ein Entleerungsbedarf angezeigt wird.

## Patentansprüche

1. Feststoff-Ausbringmodul (1) zum Abführen von Feststoffen aus Mischfluid führenden Vorrichtungen (100), insbesondere aus Zerkleinerungsvorrichtungen für Feststoffanteile in Mischfluid, mit
- einem Feststoffeinlass (3),
- Koppelmitteln (31) zum Anschluss des Feststoffeinlasses (3) an eine Mischfluid führende Vorrichtung (100),
- einem Feststoffauslass (5),
- einem Schließkörper (9), der an dem Feststoffauslass (5) angeordnet und zwischen einer Freigabestellung und einer Sperrstellung hin- und herbewegbar ist, und
- einer sich von dem Feststoffeinlass (3) zu dem Feststoffauslass (5) erstreckenden Förderkammer (7),
**gekennzeichnet durch** einen Füllstandsdetektor (13), der dazu eingerichtet ist, eine Feststoffansammlung in der Förderkammer (7) zu erfassen und anzuzeigen.

2. Feststoff-Ausbringmodul (1) nach Anspruch 1,
**gekennzeichnet durch** ein Fördermittel, welches in der Förderkammer (7) angeordnet und dazu eingerichtet ist, dem Feststoffeinlass (3) zugeführte Feststoffe zum Feststoffauslass (5) zu fördern.

3. Feststoff-Ausbringmodul (1) nach Anspruch 1 oder 2,
wobei die Förderkammer (7) unmittelbar stromaufwärts des Auslasses zur Horizontalen geneigt verläuft, insbesondere vertikal.

4. Feststoff-Ausbringmodul (1) nach einem der vorstehenden Ansprüche,
wobei der Schließkörper (9) als Absperrschieber ausgebildet ist.

5. Feststoff-Ausbringmodul (1) nach einem der vorstehenden Ansprüche,
wobei der Füllstandsdetektor (13) als Sonde ausgebildet ist, die einen Sondenkörper (15) zum Einführen in die Förderkammer (7) aufweist.

6. Feststoff-Ausbringmodul (1) nach Anspruch 5
wobei der Sondenkörper (15) ein Anzeigemittel aufweist, welches von außen sichtbar und dazu eingerichtet ist, anzuzeigen, ob der Sondenkörper (15) bis zu seiner maximalen Eindringtiefe vordringen kann oder nicht.

7. Feststoff-Ausbringmodul (1) nach Anspruch 6,
wobei das Anzeigemittel dazu eingerichtet ist, die tatsächliche Eindringtiefe des Sondenkörpers (15) anzuzeigen.

8. Feststoff-Ausbringmodul (1) nach einem der Ansprüche 2 bis 6,
wobei das Fördermittel als Volumenförderer ausgebildet ist.

9. Feststoff-Ausbringmodul (1) nach Anspruch 8,
wobei das Fördermittel einen Kolbenschieber (17) aufweist, der von dem Feststoffeinlass (3) in Richtung des Feststoffauslasses (5) bewegbar und zwischen einer eingefahrenen Stellung und einer ausgeschobenen Stellung hin- und herbewegbar ist.

10. Feststoff-Ausbringmodul nach Anspruch 9,
wobei der Kolbenschieber (17) einen Kolbenkopf (19) einen Kolbenmantel (21) aufweist, der sich umfänglich von dem Kolbenkopf (19) aus von der Förderkammer (7) fort erstreckt, wobei der Kolbenmantel (21) dazu eingerichtet ist, den Feststoffeinlass (3) zu schließen, wenn der Kolbenschieber (17) von der eingefahrenen Stellung in die ausgeschobene Stellung bewegt wird.

11. Feststoff-Ausbringmodul (1) nach einem der vorstehenden Ansprüche,
wobei der Schließkörper (9) motorisch, betätigbar ist, und wobei das Feststoff-Ausbringmodul eine elektronische Steuereinheit aufweist, die signalleitend mit dem Füllstandsdetektor (13) und dem Antrieb des Schließkörpers (9) verbunden ist.

12. Feststoff-Ausbringmodul (1) nach Anspruch 11,
wobei die elektronische Steuereinheit dazu eingerichtet ist, den Antrieb des Schließkörpers (9) zu dessen Bewegung von der Sperrstellung in die Freigabestellung anzusteuern, wenn der Füllstands-Detektor (13) mittels der signalleitenden Verbindung ein repräsentatives Signal dafür ausgibt, dass eine ausreichende Feststoffansammlung am Feststoffauslass (5) vorliegt.

13. Feststoff-Ausbringmodul (50) zum Abführen von Feststoffen aus Mischfluid führenden Vorrichtungen (100), insbesondere aus Zerkleinerungsvorrichtungen für Feststoffanteile in Mischfluid, mit
- einem Feststoffeinlass (53),
- Koppelmitteln (81) zum Anschluss an eine Mischfluid führende Vorrichtung (100),
- einem Feststoffauslass (55),
und
- einer sich von dem Feststoffeinlass (53) zu dem Feststoffauslass (55) erstreckenden Sammelkammer (57),
**gekennzeichnet durch** einen Füllstandsdetektor (63), der dazu eingerichtet ist, eine Feststoffansammlung in der Sammelkammer (57) zu erfassen und anzuzeigen.

14. Mischfluid führende Vorrichtung (100), mit
einem Sammelbereich für aus dem Mischfluid abgeschiedene Feststoffe, und
einem mit dem Sammelbereich kommunizierenden Auslass (109),
**dadurch gekennzeichnet, dass** ein Feststoff-Ausbringmodul (1, 50) an den Auslass (109) angeschlossen ist, welches nach einem der vorstehenden Ansprüche ausgebildet ist.

15. Vorrichtung nach Anspruch 14,
wobei die Vorrichtung Koppelmittel zum Anschluss des Feststoff-Ausbringmoduls (1, 50) aufweist, die mit den Koppelmitteln des Feststoff-Ausbringmoduls (1, 50) kooperieren, und vorzugsweise zum kraft- oder formschlüssigen reversibel lösbaren Koppeln ausgebildet sind.

16. Vorrichtung nach Anspruch 14 oder 15,
wobei die Vorrichtung eine Zerkleinerungsvorrichtung für Feststoffanteile in Mischfluiden ist.

17. Verwendung eines Feststoff-Ausbringmoduls (1, 50) zum Entfernen von abgeschiedenen Feststoffen aus einer Mischfluid führenden Vorrichtung (100), insbesondere aus einer Zerkleinerungsvorrichtung für Feststoffanteile in Mischfluiden,
wobei das Ausbringmodul nach einem der Ansprüche 1 bis 13 ausgebildet ist.

## Claims

1. A solids discharge module (1) for discharging solids out of devices (100) carrying mixed fluid, in particular comminuting devices for solids contents in a mixed fluid, comprising
- a solids inlet (3),
- coupling means (31) for connecting the solids inlet (3) to a device (100) carrying a mixed fluid,
- a solids outlet (5),
- a closing member (9) which is arranged at the solids outlet (5) and is reciprocable between a release position and a blocking position, and
- a conveyor chamber (7) extending from the solids inlet (3) to the solids outlet (5),
**characterised by** a filling state detector (13) adapted to detect and display a solids accumulation in the conveyor chamber (7).

2. A solids discharge module (1) as set forth in claim 1,
**characterised by** a conveyor means which is arranged in the conveyor chamber (7) and is adapted to convey solids fed to the solids inlet (3) to the solids outlet (5).

3. A solids discharge module (1) as set forth in claim 1 or claim 2
wherein the conveyor chamber (7) extends inclinedly relative to the horizontal immediately upstream of the outlet, in particular vertically.

4. A solids discharge module (1) as set forth in one of the preceding claims
wherein the closing member (9) is in the form of a shut-off slide valve.

5. A solids discharge module (1) as set forth in one of the preceding claims
wherein the filling state detector (13) is in the form of a probe having a probe body (15) for introduction into the conveyor chamber (7).

6. A solids discharge module (1) as set forth in claim 5
wherein the probe body (15) has a display means which is visible from the exterior and is adapted to display whether the probe body (15) can or cannot penetrate to its maximum penetration depth.

7. A solids discharge module (1) as set forth in claim 6
wherein the display means is adapted to display the actual depth of penetration of the probe body (15).

8. A solids discharge module (1) as set forth in one of claims 2 through 6
wherein the conveyor means is in the form of a volume conveyor.

9. A solids discharge module (1) as set forth in claim 8
wherein the conveyor means has a piston slider (17) which is moveable from the solids inlet (3) in the direction of the solids outlet (5) and is reciprocable between a retracted position and an extended position.

10. A solids discharge module (1) as set forth in claim 9
wherein the piston slider (17) has a piston head (19) and a piston casing (21) extending peripherally from the piston head (19) away from the conveyor chamber (7), wherein the piston casing (21) is adapted to close the solids inlet (3) when the piston slider is moved from the retracted position into the extended position.

11. A solids discharge module (1) as set forth in one of the preceding claims
wherein the closing member (9) is actuable by motor means and wherein the solids discharge module has an electronic control unit connected in signal-conducting relationship to the filling state detector (13) and the drive of the closing member (9).

12. A solids discharge module (1) as set forth in claim 11
wherein the electronic control unit is adapted to actuate the drive of the closing member (9) for the movement thereof from the blocking position into the release position when the filling state detector (13) by means of the signal-conducting connection outputs a representative signal that there is a sufficient solids accumulation at the solids outlet (5).

13. A solids discharge module (50) for the discharge of solids out of devices (100) carrying mixed fluid, in particular comminuting devices for solids contents in a mixed fluid, comprising
- a solids inlet (53),
- coupling means (81) for connection to a device (100) carrying a mixed fluid,
- a solids outlet (55), and
- a collecting chamber (57) extending from the solids inlet (53) to the solids outlet (55),
**characterised by** a filling state detector (63) adapted to detect and display a solids accumulation in the collecting chamber (57).

14. A device (100) carrying a mixed fluid comprising
a collecting region for solids separated from the mixed fluid, and
an outlet (109) communicating with the collecting region,
**characterised in that** connected to the outlet (109) is a solids discharge module (1, 50) as set forth in one of the preceding claims.

15. A device as set forth in claim 14
wherein the device has coupling means for connection of the solids discharge module (1, 50), that cooperate with the coupling means of the solids discharge module (1, 50) and are preferably adapted for force-locking or positively locking, reversibly releasable coupling.

16. A device as set forth in claim 14 or claim 15
wherein the device is a comminuting device for solids contents in mixed fluids.

17. Use of a solids discharge module (1, 50) for removing separated solids from a device (100) carrying mixed fluid, in particular from a comminuting device for solids contents in mixed fluids, wherein the discharge module is as set forth in one of claims 1 through 13.

## Revendications

1. Module d'extraction de matière solide (1) pour évacuer des matières solides de dispositifs guidant un fluide mixte (100), en particulier de dispositifs de broyage pour parts de matière solide dans le fluide mixte, avec
- une entrée de matière solide (3),
- des moyens d'accouplement (31) pour le raccordement de l'entrée de matière solide (3) à un dispositif guidant un fluide mixte (100),
- une sortie de matière solide (5),
- un corps de fermeture (9), qui est disposé sur la sortie de matière solide (5) et peut être animé d'un mouvement de va-et-vient entre une position de libération et une position de blocage, et
- une chambre de transport (7) s'étendant à partir de l'entrée de matière solide (3) vers la sortie de matière solide (5),
**caractérisé par** un détecteur de remplissage (13), qui est conçu pour détecter et pour afficher une accumulation de matière solide dans la chambre de transport (7).

2. Module d'extraction de matière solide (1) selon la revendication 1,
**caractérisé par** un moyen de transport, lequel est disposé dans la chambre de transport (7) et conçu pour transporter les matières solides amenées à l'entrée de matière solide (3) vers la sortie de matière solide (5).

3. Module d'extraction de matière solide (1) selon la revendication 1 ou 2,
dans lequel la chambre de transport (7) s'étend directement en amont de la sortie de manière inclinée par rapport à l'horizontale, en particulier verticalement.

4. Module d'extraction de matière solide (1) selon l'une quelconque des revendications précédentes,
dans lequel le corps de fermeture (9) est réalisé en tant que vanne d'arrêt.

5. Module d'extraction de matière solide (1) selon l'une quelconque des revendications précédentes,
dans lequel le détecteur de remplissage (13) est réalisé en tant que sonde, qui présente un corps de sonde (15) à introduire dans la chambre de transport (7).

6. Module d'extraction de matière solide (1) selon la revendication 5
dans lequel le corps de sonde (15) présente un moyen d'affichage, lequel est visible de l'extérieur et est conçu pour afficher si le corps de sonde (15) peut avancer ou non jusqu'à sa profondeur de pénétration maximale.

7. Module d'extraction de matière solide (1) selon la revendication 6,
dans lequel le moyen d'affichage est conçu pour afficher la profondeur de pénétration effective du corps de sonde (15).

8. Module d'extraction de matière solide (1) selon l'une quelconque des revendications 2 à 6,
dans lequel le moyen de transport est réalisé en tant que transporteur volumique.

9. Module d'extraction de matière solide (1) selon la revendication 8,
dans lequel le moyen de transport présente un robinet à piston (17), qui est mobile à partir de l'entrée de matière solide (3) en direction de la sortie de matière solide (5) et peut être animé d'un mouvement de va-et-vient entre une position rentrée et une position sortie.

10. Module d'extraction de matière solide selon la revendication 9,
dans lequel le robinet à piston (17) présente une tête de piston (19) une jupe de piston (21), qui s'étend sur sa périphérie à partir de la tête de piston (19) à distance de la chambre de transport (7),
dans lequel la jupe de piston (21) est conçue pour fermer l'entrée de matière solide (3) lorsque le robinet à piston (17) est déplacé à partir de la position rentrée dans la position sortie.

11. Module d'extraction de matière solide (1) selon l'une quelconque des revendications précédentes,
dans lequel le corps de fermeture (9) peut être actionné de façon motorisée, et dans lequel le module d'extraction de matière solide présente une unité de commande électronique, qui est reliée par signalisation au détecteur de remplissage (13) et à l'entraînement du corps de fermeture (9).

12. Module d'extraction de matière solide (1) selon la revendication 11,
dans lequel l'unité de commande électronique est conçue pour commander l'entraînement du corps de fermeture (9) pour le déplacement de celui-ci à partir de la position de blocage dans la position de libération, lorsque le détecteur de remplissage (13) émet, au moyen de la liaison de signalisation, un signal représentatif du fait qu'une accumulation de matière solide suffisante est présente sur la sortie de matière solide (5).

13. Module d'extraction de matière solide (50) pour l'évacuation de matières solides de dispositifs guidant un fluide mixte (100), en particulier de dispositifs de broyage pour des parts de matière solide dans le fluide mixte, avec
- une entrée de matière solide (53),
- des moyens d'accouplement (81) pour le raccordement à un dispositif guidant un fluide mixte (100),
- une sortie de matière solide (55),
et
- une chambre de collecte (57) s'étendant à partir de l'entrée de matière solide (53) vers la sortie de matière solide (55),
**caractérisé par** un détecteur de remplissage (63), qui est conçu pour détecter et pour afficher une accumulation de matière solide dans la chambre de collecte (57).

14. Dispositif guidant un fluide mixte (100), avec une zone de collecte pour les matières solides séparées du fluide mixte, et
une sortie (109) communiquant avec la zone de collecte,
**caractérisé en ce qu'**un module d'extraction de matière solide (1, 50) est raccordé à la sortie (109), lequel est réalisé selon l'une quelconque des revendications précédentes.

15. Dispositif selon la revendication 14,
dans lequel le dispositif présente des moyens d'accouplement pour le raccordement du module d'extraction de matière solide (1, 50), qui coopèrent avec les moyens d'accouplement du module d'extraction de matière solide (1, 50), et de préférence sont réalisés pour l'accouplement libérable de manière réversible à force ou par coopération de formes.

16. Dispositif selon la revendication 14 ou 15,
dans lequel le dispositif est un dispositif de broyage pour des parts de matière solide dans des fluides mixtes.

17. Utilisation d'un module d'extraction de matière solide (1, 50) pour l'élimination de matières solides séparées d'un dispositif guidant un fluide mixte (100), en particulier d'un dispositif de broyage pour des parts de matière solide dans des fluides mixtes,
dans lequel le module d'extraction est réalisé selon l'une quelconque des revendications 1 à 13.
